# EUROPÄISCHE PATENTSCHRIFT

(11) **EP 0 209 061 B1**
(45) Veröffentlichungstag und Bekanntmachung des Hinweises auf die Patenterteilung: **12.01.1994**
(21) Anmeldenummer: 86109309.4
(22) Anmeldetag: 08.07.1986
(51) Int. Cl.: C07K 7/10, C07K 1/00, A61K 37/02

(54) **Neue Polypeptide mit blutgerinnungshemmender Wirkung, Verfahren zu deren Herstellung bzw. Gewinnung, deren Verwendung und diese enthaltende Mittel**
Peptides having an anticoagulant activity, process for their preparation, obtention, their use and agents containing them
Peptides à activité anticoagulante, leur procédé de préparation, d'obtention, leur application et les agents les contenant

(30) Priorität: 17.07.1985 DE 3525428; 16.01.1986 DE 3601032
(43) Veröffentlichungstag der Anmeldung: 21.01.1987
(73) Patentinhaber: HOECHST AKTIENGESELLSCHAFT, 65926 Frankfurt am Main (DE)
(72) Erfinder: Kramer, Martin, Prof. Dr., D-6200 Wiesbaden (DE); Tripier, Dominique, Dr., D-6239 Eppstein/Taunus (DE)

(56) Entgegenhaltungen:
- EP-A- 0 158 564
- EP-A- 0 171 024
- DE-A- 3 438 296
- DE-A- 3 445 532
- DIE PHARMAZIE, Band 36, Nr. 10, Oktober 1981, Seiten 653-660, VEB Verlag Volk; P. WALSMANN et al.: "Biochemische und pharmakologische Aspekte des Thrombininhibitors Hirudin"

## Beschreibung

Antikoagulantien dienen der Prophylaxe und Therapie thrombo-embolischer Prozesse; ihr Haupteinsatzgebiet liegt dabei vor allem bei venösen Thromboembolien. Antikoagulantien werden ferner bei der Herstellung von Blutkonserven benötigt. Derivate des 4-Hydroxycumarins oder des 1,4-Indandions, die beispielsweise für diesen Zweck angewendet werden, weisen trotz weitgehender Optimierung eine Reihe von Nachteilen auf.

Es ist daher wünschenswert, besonders in der Humanmedizin Blutgerinnungshemmer zur Verfügung zu haben, die eine geringe Toxizität und wenig Nebenwirkungen aufweisen und die durch ihren Metabolismus keine Belastung des erkrankten Organismus darstellen.

Außer den körpereigenen plasmatischen Hemmstoffen wie Antithrombin III besitzen auch viele andere Proteine blutgerinnungshemmende Wirkung, wie z.B. der KunitzInhibitor, der aus Sojabohnen gewonnen wird. Dieser Hemmstoff blockt die Blutgerinnungskaskade durch Inhibition des aktivierten Faktors X, aber die Spezifität des Inhibitors ist so gering, daß viele Nebenwirkungen entstehen: Hemmung des Plasmakallikreins, des Plasmins, des Trypsins, so daß die therapeutischen Anwendungen ausgeschlossen sind. Auch andere Wirkstoffe, wie der Ascaris- oder der Kazals-Inhibitor, konnten wegen mangelnder Spezifität keine Bedeutung erlangen.

Hirudin, ein aus Hirudo medicinalis gewonnenes Polypeptid, seigt dagegen eine spezifische Antithrombin-Aktivität.

In der europäischen Patentanmeldung 0 171 024 wird nun ein gentechnologisches Verfahren zur Herstellung von speziellen Hirudinen beschrieben. Ferner beschreibt die europäische Patentanmeldung 0 158 564 Expressionsvektoren zur Klonierung und Expression von Hirudin und Hirudinanaloga.

Das aufwendige Verfahren zu seiner Isolierung und Reinigung hat sich bisher nachteilig auf seine praktische Anwendung ausgewirkt.

Es wurde nun gefunden, daß sich aus Blutegeln hochaktive Polypeptide der Formel I isolieren lassen.

Die Erfindung betrifft daher Polypeptide der Formel I 
1 2 3 4 5 6 7 8 9 10 11 12 13 14
H-(X)m-A-B-C-Tyr-Thr-Asp-Cys-F-Glu-Ser-Gly-Gln-Asn-Leu-
15 16 17 18 19 20 21 22 23 24 25 26 27
-Cys-Leu-Cys-Glu-Gly-Ser-Asn-Val-Cys-Gly- J -Gly-Asn-
28 29 30 31 32 33 34 35 36 37 38 49 40
-Lys-Cys-Ile-Leu-Gly-Ser-Asp-Gly- D - G -Asn-Gln-Cys-
41 42 43 44 45 46 47 48 49 50 51 52 53
-Val-Thr-Gly-Glu-Gly-Thr-Pro-Lys-Pro-Gln-Ser-His-Asn-
54 53 56 57 58 59 60 61 62 63 64 65
-Asp-Gly-Asp-Phe-Glu- E -Ile-Pro-Glu-Glu-Tyr(R)- H -
66
-Gln-(Z)n-OH

(I)
in welcher
- m =: 0 - 50,
- n =: 0 - 100 und
- R: phenolischen Wasserstoff oder eine Phenolestergruppe bedeuten,
- X: für gleiche oder verschiedene Reste natürlich vorkommende α-Aminosäuren steht,
- Z: für gleiche oder verschiedene Reste natürlich vorkommender α-Aminosäuren steht und
- A: für Ile oder die Abwesenheit einer Aminosäure steht
- B: für Ile oder Thr oder die Abwesenheit einer Aminosäure steht,
- C: Thr, Val, Ile, Leu oder Phe,
- D: Glu oder die Abwesenheit einer Aminosäure
- E: Glu oder Pro,
- F: Thr oder Ile bedeuten,
- G: Lys oder Lys-Asp bedeutet,
- H: Ala oder Leu bedeutet und
- J: Glu oder Lys bedeuten,
in der die 6 Cys-Reste paarweise über Disulfid-Brücken verknüpft sind,
sowie deren physiologisch verträgliche Salze, wobie wenn
J = Gln
G ≠ Lys oder H ≠ Leu oder R ≠ Wasserstoff uns falls A und B abwesend sind C ≠ Val.

Die drei Disulfid-Brücken befinden sich vorzugsweise zwischen den Cys-Resten in den Positionen 7 und 15, 17 und 29 sowie 23 und 40.

Natürlich vorkommende α-Aminosäuren sind insbesondere Gly, Ala, Ser, Thr, Val, Leu, Ile, Asp, Asn, Glu, Gln, Cys, Met, Arg, Lys, Hyl, Orn, Cit, Tyr, Phe, Trp, His, Pro und Hyp.

R bedeutet vorzugsweise Wasserstoff, SO₃E oder PO₃H₂; besonders bevorzugt ist Wasserstoff.

Als Salze kommen insbesondere Alkali- und Erdalkalisalze, Salze mit physiologisch verträglichen Aminen sowie Salze mit physiologisch verträglichen Säuren, wie HCl, H₂SO₄, Maleinsäure oder Essigsäure in Frage.

Bevorzugt sind Polypeptide der Formel I, in denen C für Thr steht; ferner solche, bei denen C für Thr und A für Ile steht. Speziell geeignete Peptide sind solche mit
- A= Ile, B= Thr, C= Thr, D= Glu, E= Glu, F= Thr, G= Lys, H= Leu, J= Gln, m= null, n= null, R= H oder SO₃H;
- m= null, n= null, R= H oder SO₃H, A= Ile, B= direkte Bindung, C= Thr, D= Glu, E= Glu, F= Thr, G= Lys, H Leu, J= Gln;
- m= null; n= null; R= H oder SO₃H, A= Ile, B= direkte Bindung, C= Thr, D= Glu, E= Glu, F= Ile, G= Lys, H= Leu, J= Gln;
- m= null, n= null, R= H oder SO₃H, A= Ile, B= direkte Bindung, C= Thr, D= Glu, E= Pro, F= Thr, G= Lys, H= Leu, J= Gln;
- m= null, n= null, R= H oder SO₃H, A= Ile, B= direkte Bindung, C= Thr, D= Glu, E= Pro, F= Ile, G= Lys, H= Leu, J= Gln;
- m= null, n= null, R= H oder SO₃H, A= direkte Bindung, B= direkte Bindung, C= Thr, D= direkte Bindung, E= Glu, F= Thr, G= Lys, H= Leu, J= Gln;
- A= Ile, B= direkte Bindung, C= Thr, D= direkte Bindung, E= Glu, F= Ile, G= Lys-Asp, H= Ala, J= Lys, m= null, n= null, R= SO₃H;
- A= Ile, B= direkte Bindung, C= Thr, D= direkte Bindung, E= Glu, F= Ile, G= Lys-Asp, H= Ala, J= Lys, m= null, n= null, R= Wasserstoff.

Die Erfindung betrifft auch die neuen biologisch aktiven peptidischen Spaltprodukte, die durch chemische oder enzymatische Spaltung dieser Polypeptide erhalten werden mit Ausnahme von Spaltprodukten eines Peptids der Formel (niche EP-A-0 158 564): 
Ile Thr Tyr Thr Asp Cys Thr Glu Ser Gly Gln Asn Leu Cys Leu Cys Glu Gly Ser Asn Val
Cys Gly Lys Gly Asn Lys Cys Ile Leu Gly Ser Asn Gly Lys Gly Asn Gln Cys Val Thr Gly
Glu Gly Thr Pro Asn Pro Gln Ser His Asn Asn Gly Asp Phe Glu Glu Ile Pro Glu Glu Tyr
Leu Gln
Die Erfindung betrifft weiterhin ein Verfahren zur Gewinnung eines gereinigten Polypeptids der obengenannten Formel, das dadurch gekennzeichnet ist, daß man das Polypeptid aus Würmern des Stammes Annelida mit Hilfe einer Kombination von Extraktionsmethoden, Fällungsmethoden, Membranfiltration und/oder chromatographischen Verfahren isoliert, eine gegebenenfalls vorhandene Phenolestergruppe R gewünschtenfalls hydrolytisch unter Bildung der phenolischen Hydroxylgruppe abspaltet und das erhaltene Peptid gegebenenfalls in seine physiologisch verträglichen Salze überführt.

Das Polypeptid wird vorzugsweise aus den Halsdrüsen von Würmern der Klasse Hirudinea, insbesondere aus solchen der Ordnung Gnathobdellida gewonnen. Bevorzugt sind die Gattungen Hirudo, Gnathobdella, Haemadipsa und Philaemon. Besonders bevorzugt ist Hirudo medicinalis. Neben den Halsdrüsen des Blutegels kann auch dessen vordere Körperregion oder der ganze Blutegel Verwendung finden.

Ein Verfahren zur Gewinnung eines Rohextraktes aus Blutegeln ist in Enzymology, Band 5 "Hirudin as an Inhibitor of Thrombin" beschrieben. Ein Reinigungsverfahren für Hirudin ist aus Bull. Soc. Chim. Biol. 45 (1963) 55 bekannt.

Beim erfindungsgemäßen Verfahren hat sich insbesondere eine Kombination von Fällungsmethoden und von Gel-Permeations-Chromatographie oder Ultrafiltration, Affinitätschromatographie und von hoch auflösender Verteilungschromatographie an "Reverse-Phase"-Material und Chromatographie an Kieselgel oder Aluminumoxid als nützlich erwiesen. Je nach Beschaffenheit des Rohextraktes können aber auch andere Chromatographie-Verfahren vorteilhaft angesetzt werden (evtl. auch in Kombination mit dem obengenannten Verfahren), wie z.B. Kationen- oder AnionenAustausch-Chromatographie, Chromatographie an unspezifischen Absorbentien, insbesondere Hydroxylapatit.

Um einen für die Chromatographie geeigneten Rohextrakt zu gewinnen, kann man die Blutegel in der bei Bagdy et al. Methods of Enzymology 45 [1976] 669-678 beschriebenen Weise aufarbeiten. Man kann aber auch beispielsweise die Kopfteile des Blutegels in gefrorenem Zustand zerkleinert und mittels einer wäßrigen Puffer-Lösung (z.B. Phosphatpuffer) extrahiert werden. Das unlösliche Material wird z.B. durch kurzes Zentrifugieren oder durch Filtration über Gaze abgetrennt und das Polypeptid aus dem so erhaltenen Extrakt abgetrennt und isoliert. Es ist vorteilhaft, diesen Extrakt schnell auf 70° bis 90°C zu erhitzen, weil dadurch die Hauptmenge der proteolytischen Enzyme denaturiert und ausfällt, deren Abtrennung dann z.B. durch Zentri-fugation erfolgen kann. Man isoliert aus dem Extrakt die Proteinfraktion, die das erfindungsgemäße Peptid enthält, z.B. durch Fällung in der Weise, daß man den Extrakt in ein wassermischbares organisches Lösungsmittel gibt. Z.B. kann Aceton in einer mehrfachen Menge des Extraktvolumen, vorzugsweise der etwa 10-fachen Menge eingesetzt werden, wobei die Fällung in der Kälte, üblicherweise bei 0 bis -40°C, vorzugsweise bei etwa -20°C vorgenommen wird.

Eine andere Möglichkeit, die Fällung durchzuführen, ist die Zugabe von Salzen, wie z.B. Ammoniumsulfat. Durch pH-Steuerung erreicht die Fällung eine gewisse Selektivität. Die erfindungsgemäßen Peptide, die isoelektrische Punkte von 3,5 - 4 haben, können im pH-Bereich zwischen 3 und 5, vorzugsweise etwa 4, durch Zugabe von Ammoniumsulfat bis zu einer Konzentration von etwa 50 % ausgefällt werden, wobei eine Vielzahl von Begleitproteinen dabei in der Lösung bleibt. Auch diese Fällung wird unter Kühlung bei etwa -5 bis +15°C vorzugsweise zwischen 0 und +4°C durchgeführt.

Aus diesem Rohextrakt können Proteine mit höherem Molekulargewicht z.b. durch Ultrafiltration oder durch Gel-Permeations-Chromatographie abgetrennt werden. Bei größeren Ansätzen kann die Ultrafiltation z.B. In zwei Stufen erfolgen: In der ersten Stufe arbeitet man mit einer Kapillarmembran mit einer Ausschlußgrenze von 50 000 Dalton und dann in der zweiten Stufe mit einer Flachmembran mit einer Ausschlußgrenze von 10 000 Dalton. Mit Hilfe der Kapillarmembran erreicht man eine schnelle Abtrennung von höhermolekularem Material, welches den Durchfluß durch die selektiv arbeitende Flachmembran verhindern würde. Bei kleinen Mengen kann man auch auf die erste Stufe der Ultrafiltation verzichten.

Eine Reinigung des Rohextraktes kann auch mittels Ionenaustausch-Chromatographie, beispielsweise an DEAE- ^{(R)}Sephadex, in der von Markwardt, Walsmann, Hoppe-Seyler's Z. Physiol. Chem. 348 [1967] 1381-1386 beschriebenen Weise erfolgen.

Das so erhaltene Material besteht aus einer Mischung der erfindungsgemäßen Thrombininhibitoren und anderen Polypeptiden. Ein bevorzugtes Verfahren zur Gewinnung der Inhibitoren der Formel I mit R= H bzw. SO₃H besteht darin, daß die Thrombininhibitoren aufgrund der Eigenschaften der Komplexbildung mit am Träger gebundenen Thrombin von Produkten, die keine Komplexe mit Thrombin bilden, getrennt werden. Die Fraktionen, die aufgrund ihrer Thrombinaffinität gewonnen worden sind, können wiederum durch ein zweites hochauflösendes chromatographisches System In einzelne Komponenten aufgelöst werden. So werden die Inhibitoren der Formel I isoliert. Für die Affinitätschromatographie hat sich die Benützung von ThrombinSepharose besonders bewährt. Thrombin-Sepharose wurde nach dem Verfahren von Brosstad (Thrombos Res. II, 119, 1977) hergestellt.

Für die Trennung schüttet man Thrombin-Sepharose in eine Säule mit einem geeigneten Puffer, wie z.B. 0,1 M N-Methylmorpholinacetatpuffer pH 8,0 oder Tris/HCl 0,1 M pH 8,5 . Nach Äquilibrierung der Säule wird das Gemisch aus der Fällung in gleichem Puffer gelöst und auf die Säule aufgebracht. Die Peptide, die keine Thrombinaffinität haben, werden durch Spülung mit dem Puffer entfernt. Danach wird der Komplex Thrombin/Thrombin-Inhibitor durch Spülung der Säule mit einem Puffer aus 0,5 - 2 M Benzamidin oder 4-Amino-Benzamidin in 0,1 M N-Methylmorpholinacetat pH 8,0 aufgelöst. Die verschiedenen aktiven Fraktionen werden zusammen gepoolt und durch übliche Gel-PermeationsChromatographie auf Sephadex G 25 mit 0,05 M N-Methylmorpholinacetat pH 8,0 entsalzt.

Die Trennung der verschiedenen Thrombin-Inhibitoren voneinander wird durch hoch auflösende chromatographische Verfahren besorgt. Dafür hat sich besonders die HPLC bewährt.

Durch das hohe Auflösungsvermögen der HPLC-Technologie ist es möglich, die Inhibitoren der Formel I voneinander und von geringfügigen Mengen von Begleitprotein zu trennen und rein darzustellen.

Für die stationäre Phase haben sich derivatisierte Kieselgele mit geeigneter Korngröße (z.B. zwischen 3 und 20 µm) vorteilhaft erwiesen. Für die Derivatisierung des Kieselgels eignen sich neben den verbreiteten Octadecylsilanresten auch eine Vielzahl anderer Silanreste oder deren Mischungen, wie Silanreste mit niedrigem Alkyl, Phenylalkyl- oder amino substituiertem Alkyl, wobei letztere eine gewisse Kombination von Ionenaustausch- und "Reverse-Phase" Chromatographie bieten. Es können beispielsweise Trennsäulen von 5 bis 25 cm Länge und einem Durchmesser von 3 bis 10 mm verwendet werden. Als gepuffertes Elutionsmittel kommen alle sekundären oder tertiären Mischungen zwischen Wasser, organischen Lösungsmitteln geeigneter Lipophilie in Frage, wie z.B. niedrige Alkohole, Ketone, Nitrile, Ether, Säure, Amine, Glykolether, Amide und deren Derivate. Als Puffersubstanz können organische und anorganische Salze oder andersartige Zusätze verwendet werden. Die Elution erfolgt vorteilhaft bei einem pH-Wert zwischen 2 und 8.

Die Benutzung von flüchtigen Puffersubstanzen, wie Ammoniumacetat oder Ammoniumhydrogencarbonat, erlaubt die Gewinnung der Inhibitoren aus dem Eluat durch einfache Gefriertrocknung.

Die Abspaltung einer Sulfatmonoestergruppe R in Position 64 kann in Analogie zu der in DE-A-33 42 139 beschriebenen Weise sauer katalysiert oder ensymatisch mit Hilfe einer Arylsulfatase erfolgen.

Die erfindungsgemäßen Polypeptide der Formel I sind farblos, in Wasser und in wäßrigen Puffern löslich, zeigen sich homogen in der Polyacrylamid-Elektrophorese und besitzen isoelektrische Punkte von 3,5 bis 4(bestimmt durch isoelektrische Fokussierung). Bestimmt man die Aminosäurezusammensetzung nach der Methode von Moore und Stein (Methods of Enzymology Band VI, 819 - 831, herausgegeben von Rolovick und Kaplan, Academic Press, New York, London, 1963), findet man die in Tabelle 1 angegebenen Werte

**Tabelle 1**

| | II | III | IV | V | VI | VII | VIII | IX |
|---|---|---|---|---|---|---|---|---|
| Asparaginsäure | 9 | 9 | 9 | 9 | 9 | 9 | 10 | 10 |
| Threonin | 6 | 5 | 4 | 5 | 4 | 5 | 4 | 4 |
| Serin | 4 | 4 | 4 | 4 | 4 | 4 | 4 | 4 |
| Glutaminsäure | 13 | 13 | 13 | 12 | 12 | 12 | 11 | 11 |
| Prolin | 3 | 3 | 3 | 4 | 4 | 3 | 3 | 3 |
| Glycin | 9 | 9 | 9 | 9 | 9 | 9 | 9 | 9 |
| Valin | 2 | 2 | 2 | 2 | 2 | 2 | 2 | 2 |
| Cystein | 6 | 6 | 6 | 6 | 6 | 6 | 6 | 6 |
| Isoleucin | 3 | 3 | 4 | 3 | 4 | 2 | 4 | 4 |
| Leucin | 4 | 4 | 4 | 4 | 4 | 4 | 3 | 3 |
| Tyrosin | 2 | 2 | 2 | 2 | 2 | 2 | 2 | 2 |
| Phenylalanin | 1 | 1 | 1 | 1 | 1 | 1 | 1 | 1 |
| Lysin | 3 | 3 | 3 | 3 | 3 | 3 | 4 | 4 |
| Histidin | 1 | 1 | 1 | 1 | 1 | 1 | 1 | 1 |
| Alanin | 0 | 0 | 0 | 0 | 0 | 0 | 1 | 1 |
| IX wird erhalten durch Desulfatisierung von VIII. | | | | | | | | |

Die Erfindung betrifft ferner ein Verfahren zur Herstellung eines Polgpeptids der obengenannten Formel I, das dadurch gekennzeichnet ist, daß man
a) es in an sich bekannter Weise mittels Festphasensynthese herstellt oder
b) zur Herstellung eines Polypeptids, in welchem m= 0 ist,
   I. Hirudin einem zweifachen Edman-Abbau unterwirft,
   II. das so erhalten Peptid mit einem Aktivester einer Aminosäure oder eines Peptids der Formel

      U-(X)ₘ-A-B-C-OH,

      in welcher m, X und A, B, C wie oben definiert sind und U für eine säuren- oder basenlabile Urethanschutz gruppe steht, umsetzt,
   III. die Phenylthiocarbamoyl-Gruppe an der ε-Amino-Funktion von Lys mittels Hydrazin
   IV. und die Urethan-Schutzgruppe U mit Hilfe einer Säure oder Base abspaltet und
   das nach a) oder b) erhaltene Polypeptid gegebenenfalls in sein physiologisch verträgliches Salz überführt.

Bei der Festphasensynthese (vgl. hierzu Atherton, Sheppard, Perspectives in Peptide Chemistry, Karger Basel 1981, Seiten 101 - 117) kann in der Regel auf eine OH-Schutzgruppe für Thr verzichtet werden.

Die Synthese des Polypeptids der Formel I erfolgt z.B. schrittweise an hydroxpethyliertem Polystyrol-Harz. Das Polystyrol ist mit beispielsweise 1 % Divinylbenzol quervernetzt. Es liegt gewöhnlich in Form kleiner Kügelchen vor.

Die Aminosäuren werden N-terminal geschützt eingesetzt. Die erste N-geschützte Aminosäure wird per Esterbildung am Träger angebracht. Nach Beseitigung der Aminoschutzgruppe wird die nächste N-geschützte Aminosäure unter Verwendung eines Kupplungsreagenses wie Dicyclohexylcarbodiimid angeknüpft. Entschützen und Zufügen weiterer Aminosäuren wird fortgesetzt, bis die gewünschte Sequenz erreicht ist.

Die Auswahl der Schutzgruppen richtet sich nach den Aminosäuren und Kupplungsmethoden.

Als Aminoschutsgruppen kommen z.B. die bekannten urethanischen Schutzgruppen wie Benzyloxycarbonyl(Z), p-Methoxycarbobenzoxy, p-Nitrocarbobenzoxy, t-Butyloxycarbonyl(Boc), Fmoc und ähnliche in Frage.

Die Boc-Gruppe wird bevorzugt, da sie unter relativ milden Bedingungen (z.B. mit Trifluoressigsäure oder HCl in organischen Lösungsmitteln) abspaltbar ist.

Threonin kann als Benzylether blockiert werden und die ε-Aminofunktion des Lysins als Z-Derivat. Diese beiden Schutzgruppen sind gegen die Abspaltungsreagenzien für die Boc-Gruppe weitestgehend resistent und können hydrogenolytisch mit einem Hydrierkatalysator (Pd/Aktivkohle) oder z.B. mit Natrium in flüssigem Ammoniak entfernt werden.

Das geschützte Peptid kann z.B. mit Hydrazin vom Harz genommen werden. Dabei entsteht das Hydrazid, welches z.B. mit N-Bromsuccinimid nach Int. J. Pept. Prot. Research 17 (1981) 6 - 11 in die freie Carbonsäure über-führt werden kann. Falls erforderlich, müssen die Disulfid-Brücken oxidativ geschlossen werden (vgl. König, Geiger, Perspectives in Peptide Chemistry, Karger Basel Seiten 31 - 44).

Bei der Verfahrensvariante b) unterwirft man Hirudin einem sweifachen Edman-Abbau, indem man dieses Polypeptid in einer geeigneten Pufferlösung, wie Pyridin/Wasser oder Dioxan/Wasser gegebenenfalls unter Zusatz einer Base wie Dimethylbenzylamin (DMBA), Dimethylallylamin (DMAA) oder Triethylamin, vorzugsweise bei etwa 50°C und einem pH-Wert von 8 - 9 mit einem Isothiocyanat, vorzugsweise Phenylisothiocyanat umsetst. Nach Entfernung des überschüssigen Puffers und des überschüssigen Phenylisothiocyanats wird das N-terminale Valin durch Behandeln mit einer Säure (Heptafluorbuttersäure oder Trifluoressigsäure) während 10 Minuten bei 50°C als Phenylthiazolinon abgespalten. Man wiederholt diese Reaktionsfolge zur Spaltung des zweiten Valins am N-Terminus.

Das auf diese Weise erhaltene Des-(Val)₂-Hirudin-Derivat wird mit einem Aktivester einer Aminosäure oder eines Peptids der Formel U-(X)ₘ-A-B-C-OH, umgesetzt. Geeignet sind z.B. p-Nitrophenyl-, Cyanomethyl-, N-Hydroxyphthalimid oder insbesondere N-Hydroxysuccinimid-Ester. Geeignete Urethanschutzgruppen U sind solche, die sauer oder alkalisch abspaltbar sind, wie z.B. Boc oder Msc. Falls erforderlich, so können auch evtl. vorhandene Funktionen in den Seitenketten von B und C durch geeignete Schutzgruppen vorübergehend geschützt werden.

Die auf diese Weise erhaltene geschützte Vorstufe des Polypeptids der Formel I (m=O) wird zur Abspaltung der phenylthiocarbamoyl-Gruppe am Lysin in einem geeignetem Lösungsmittel, wie einem niedrigen Alkohol oder dessen Gemisch mit Wasser mit Hydrazin-Hydrat behandelt.

Man spaltet aus diesem Polypeptid nun noch die restliche(n) Schutzgruppe(n) in geeigneter Weise ab (Boc z.B. mit Trifluoressigsäure, Msc mit einer Base) und erhält so das erfindungsgemäße Polypeptid der Formel I.

Die erfindungsgemäßen Polypeptide sind spezifische stöchiometrische Hemmer des Thrombins. Die quantitative Messung der Thrombin-Hemmung durch die erfindungsgemäßen Inhibitoren zeigte, daß der Komplex Thrombin-Inhibitor/Thrombin praktisch nicht dissoziiert. Mit Hilfe dieser Meßmethode kann während der Aufarbeitung und Reinigung die Aktivität und damit der Reinheitsgrad der erfindungsgemäßen Polypeptide bestimmt werden. Die so gereinigten Polypeptide der obengenannten Formel I können dabei eine Thrombinhemmung von über 10 000 antithrombine units/mg aufweisen und damit die des herkömmlichen Hirudins übertreffen. Dabei sind in vivo die Verbindungen der Formel I mit freiem phenolischem Wasserstoff in Position 64 in der Regel noch aktiver.

Die Erfindung betrifft daher auch die Verwendung von Polypeptiden der Formel I, in welcher m, n, R, X, Z, A, B, C, D, E, F,G, J und H die oben genannte Bedeutung haben als Blutgerinnungshemmer zur Anwendung bei der Therapie thromboembolischer Prozesse sowie deren Anwendung als Diagnostika und Reagentien.

Die Erfindung betrifft weiterhin Mittel, die ein Polypeptid der Formel I oder dessen peptidisches Spaltprodukt in einem pharmazeutisch unbedenklichen Träger enthalten.

Die erfindungsgemäßen Verbindungen können parenteral oder topisch in entsprechender pharmazeutischer Zubereitung verabreicht werden.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren, Isotoniemittel, Konservierungsstoffe oder weitere Hilfsstoffe in Lösung, Suspension oder Emulsion gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propandiol, oder Glycerin, daneben auch Zuckerlösungen wie Glucose- oder Mannitlösungen, oder auch eine Mischug aus den verschiedenen genannten Lösungen. Bei subkutaner Anwendung weisen Verbindungen der Formel I (R= phenolischer Wasserstoff) in der Regel den Vorteil einer langsameren Resorption und damit einer retardierten Wirkung auf.

Die topischen Trägerstoffe können organische oder anorganische Verbindungen sein. Typische pharmazeutisch gebrauchte Trägerstoffe sind wäßrige Lösungen, die z.B. Puffersysteme oder isotonische Mischungen von Wasser und wassermischbaren Lösungsmitteln sind, wie z.B. Alkohole oder Arylalkohole, öle, Polyalkylenglykole, Ethylcellulose, Carboxymethylcellulose, Polyvinylpyrrolidon oder Isopropylmyristat. Geeignete Puffersubstanzen sind z.B. Natriumborat, Natriumphosphat, Natriumacetat oder auch Gluconatpuffer. Die topische Anwendungsform kann auch nicht toxische Hilfsstoffe enthalten wie z.B. emulgierende Konservierungsstoffe, Vernetzer, wie z.B. Polyethylenglykole und antibakterielle Verbindungen.

### Beispiel 1

### Bestimmung der Inhibitor-Konzentration durch Thrombin-Titration

10 bis 100 µl der Inhibitor-Lösung mit einem vorher bestimmten Proteingehalt werden mit 200 µl Natriumhydrogencarbonat-Lösung (pH= 7,0; 0,5 M) versetzt. Man addiert 0,1 ml Fibrinogen-Lösung (0,5 bis 1 %) oder verdünntes Citrat-Plasma; in regelmäßigem Abstand, unter Rühren, bei Zimmertemperatur, wird ein aliquoter Teil (50 - 100 µl) der Thrombin-Lösung sugefügt (ca. 100 NIH Einheiten pro ml). Als Umschlagpunkt kann beim halbquantitativen Arbeiten das Gerinnen der Flüssigkeit innerhalb des gewählten Zeitabstandes dienen, oder, für quantitative Bestimmungen, die Turbimetrie-Messung bei 546 nm.

### Beispiel 2

Es werden freilebende Blutegel (keine Zuchttiere) der Art Hirudo medicinalis verwendet, die in Deutschland gesammelt worden sind.

Ca. 150 - 200 g gefrorene Blutegelvorderteile werden in einem Mixer mit 2 l eiskalter 0,09 %iger wäßriger Natriumchlorid-Lösung und 10 ml Octanol innerhalb von 3 Minuten homogenisiert. Nach 30 minütiger Zentrifugation bei 0°C und 10 000 Upm wird der Überstand mittels Filtration über 2 Lagen Gaze weitergeklärt und anschließend unter Rühren innerhalb von 15 Minuten auf 80°C erhitzt. Die entstandene Fällung wird durch Filtration über 4 Lagen Gase getrennt. Das Filtrat wird durch Rühren in einem Eisbad schnell auf 4°C heruntergekühlt und in 7,5 l vorgekühltes Aceton (-20°C) gegeben. Es entsteht erneut eine Fällung, die nach 5 Minuten auf einer Glasfilternutsche abfiltriert und mit 1 l kaltem Aceton (-20°C) nachgewaschen wird. Nach Trocknung im Vakuum entsteht 520 mg leicht gelbliches Pulver mit einem Proteingehalt von 62 % (bestimmt nach der Methode von Lowry). Die Antithrombinaktivität beträgt ca. 400 Einheiten pro mg.

### Beispiel 3

520 mg Pulver gemäß Beispiel 2 werden in 75 ml Wasser gelöst, dann mit 5 N Ammoniak auf pH 8,0 eingestellt und 1 Stunde bei 0 - 4°C gerührt. Der unlösliche Anteil wird innerhalb von 30 Minuten mit einer Becher-Zentrifuge bei 5000 Upm abgeschleudert. Nach Einstellung des Proteingehaltes durch Wasserzugabe auf 25 mg/ml (Lowry), wird die Lösung mit 35 ml gesättigter Ammoniumsulfat-Lösung versetzt und 1 Stunde bei 4°C gerührt. Der erste Niederschlag wird schnell durch Zentrifugation (5000 Upm/30 Minuten) abgetrennt. Man löst noch ca. 26 g Ammoniumsulfat dazu und stellt den pH-Wert mit Eisessig auf pH 4 ein. Nach 5-stündigem Stehen wird die gesamte Suspension sentrifugiert und der erhaltene Feuchtniederschlag wie folgt weiterverarbeitet.

### Beispiel 4

Der gemäß Beispiel 3 erhaltenen Feuchtniederschlag wird in 200 ml 0,1 M Ammoniumhydrogencarbonat-Lösung vom pH 8 gelöst und in einer 250 ml Amicon^{R}-Zelle mit einer 5PM 10-Flachmembran (Ausschlußgrenze 10 000 Dalton) ultrafiltriert. Dabei wird die Lösung auf ca. 40 ml eingeengt, wobei gegen Ende zweimal 150 ml 0,1 M Ammoniumhydrogencarbonat-Lösung vom pH 8,0 nachgefüllt werden. Gefriertrocknung des Rückstandes ergibt ca. 350 mg Material mit einem Proteingehalt von 89 %.

### Beispiel 5

Man schüttet eine Säule (0,9 x 15 cm) mit ThrombinSepharose in 0,1 m Tris-Puffer (HCl) pH 8.
Die Substanz aus Beispiel 3 wird im gleichen Puffer gelöst, und die Säule wird mit dieser Probe beschickt. Durch Spülung der Säule mit dem Equilibrierungspuffer werden inaktive Begleitsubstanzen entfernt. Danach mit einer Lösung von Benzamidin (1,5 M Tris Puffer pH 7) oder mit 4-Aminobenzamidin (0,2 M Tris Puffer pH 7) läßt sich das Hirudin von dem Komplex Thrombin-Hirudin verdrängen und wird portionsweise eluiert. Zum Testen der Antithrombinaktivität muß zuerst der kompetitive Inhibitor durch Gel-Filtration ^{(R)}Sephadex G 20 vom Hirudin getrennt werden. Gewicht-Ausbeute 55 %;
Aktivität 6000 bis 12 000 ATU/mg.

### Beispiel 6

20 mg Inhibitor gemäß Beispiel 3 werden in 200 µl Wasser von pH 2,16 (eingestellt mit Trifluoressigsäure + 5 % Acetonitril) gelöst und auf eine mit OctadecylsilanKieselgel (5 µm) gefüllte Stahl-Säule eingespritzt (Shandon ^{R}ODS). Die Säule wird durch einen Gradienten von maximal 2 %/Minute zwischen dem Startpuffer (Wasser-pH= 2,16 + 5 % Acetonitril) und Acetonitril eluiert. Die Fraktionen werden einzeln gesammelt. Nach Trocknung haben die erfindungsgemäßen Inhibitoren der Formel I (R= H bzw. SO₃R) eine spezifische Aktivität, die der Stöchiometrie eines 1 : 1-Komplexes mit Thrombin entspricht.

### Beispiel 7

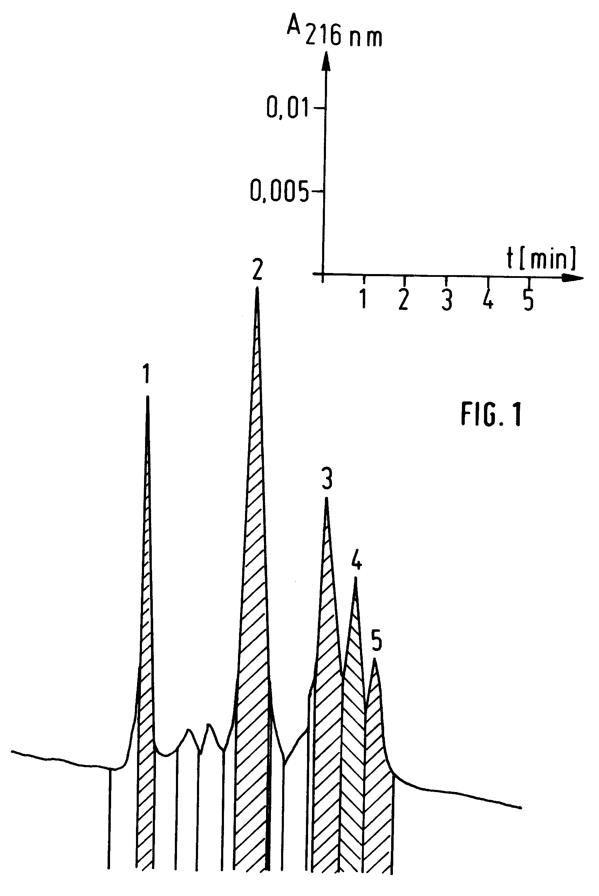

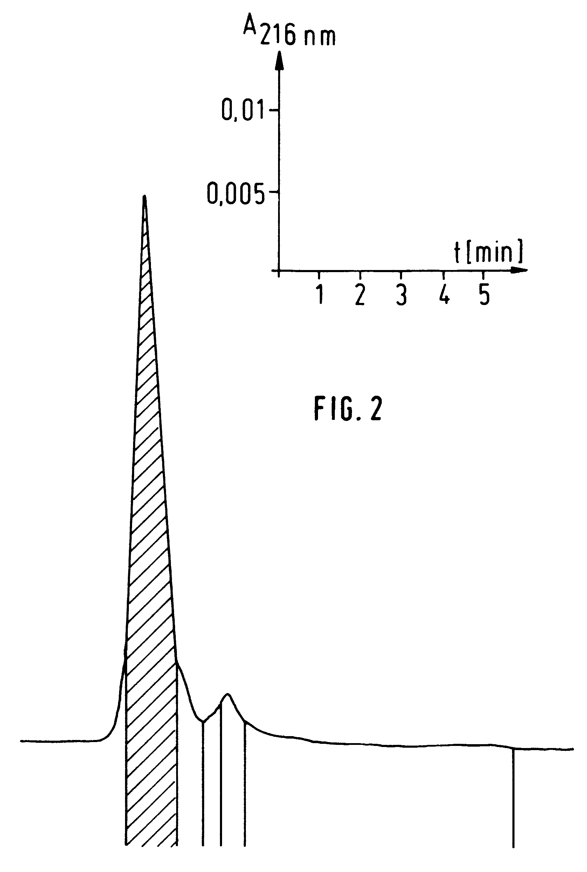

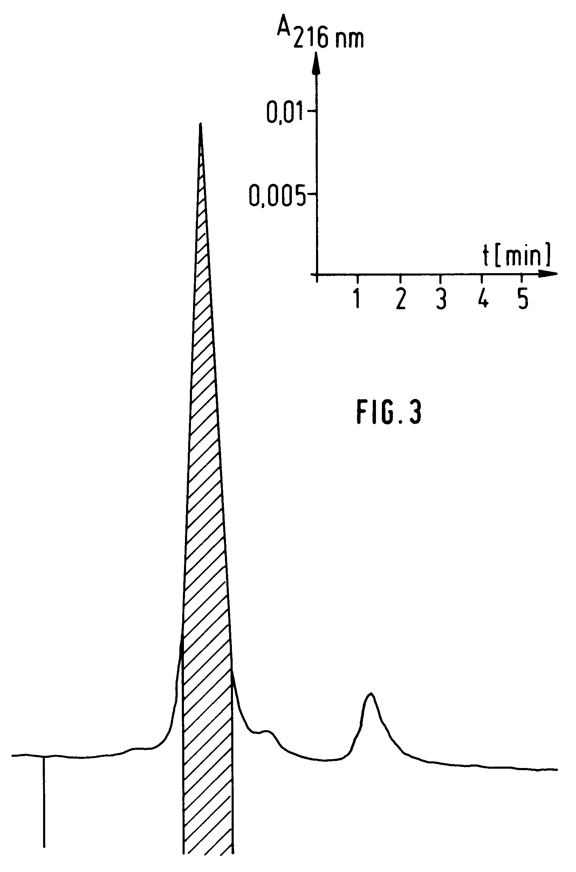

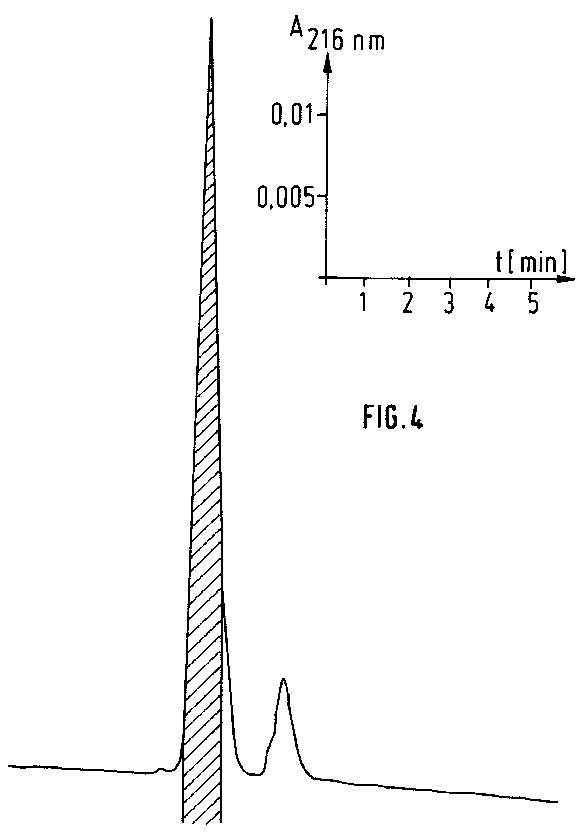

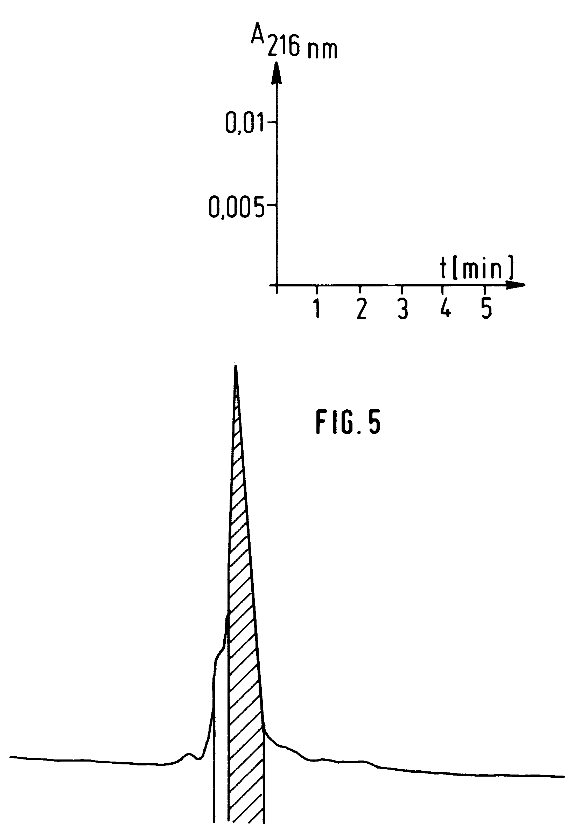

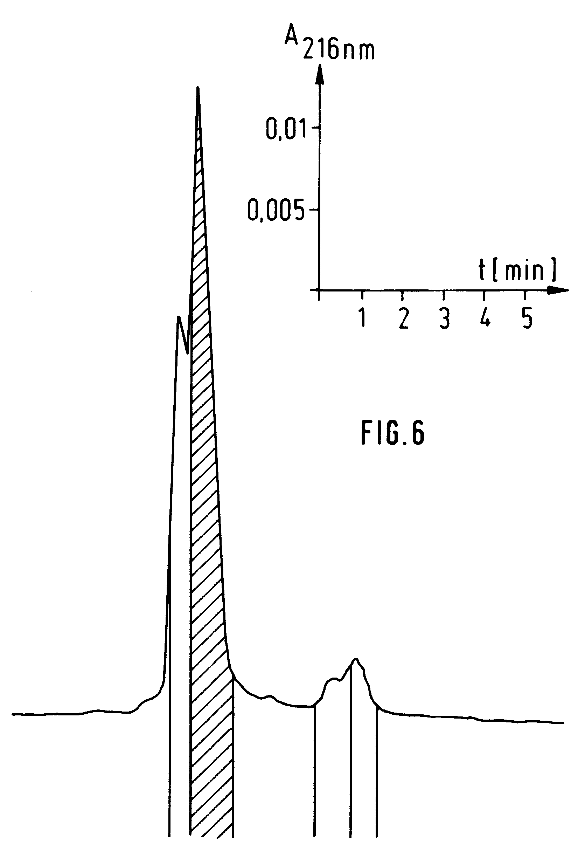

a) Das gemäß Beispiel 4 erhaltene Material wurde in der bei Markwardt, Walsmann, Hoppe-Seyler's z. Physiol. Chem. 348 [1967] 1381-1386 beschriebenen Weise an DEAE-^{(R)}Sephadex A-25 gereinigt.
b) 1,1 mg der so erhaltenen Proteinfraktion wurde mittels HPLC auf einer 25 cm x 4,6 mm ^{(R)}Bio-Rad (Richmond, CA) Hi-Pore-Säule aufgetrennt, die mit C-18 Reverse-Phase Kieselgel mit 5µ Teilchengröße und 330 Å Porenweite gefüllt war. Als mobile Phase wurde 10 % Acetonitril in Wasser mit 0,1 % Trifluoressigsäure (A) / 10 % Wasser in Acetonitril mit 0,1 % Trifluoressigsäure (B) mit einer Gradientengeschwindigkeit A:B von 1 %/Minute verwendet. Der zeitliche Verlauf der Chromatographie wurde durch Detektion bei 216 nm überwacht (siehe Fig. 1). Die in Fig. 1 schraffierten Fraktionen 1-5 wurden nochmals rechromatographiert, wobei das gleich HPLC-System benutzt wird.
c) Rechromatographie von Fraktion 1 (Fig. 2) lieferte ca. 200 µg eines Proteins, dessen Struktur durch Sequenzanalyse bestimmt wurde. Ihm kommt die folgende Struktur zu: 
ITYTDCTESGQNLCLCEGSNVCGQGNKCIL
GSDGEKN QCVTGEGTPKPQSHNDGDFEEI-
PEEYLQ.
d) Rechromatographie von Fraktion 2 (Fig. 3) lieferte ca. 300 µg eines Proteins, dessen Struktur durch Sequenzanalyse bestimmt wurde. Ihm kommt die folgende Struktur zu: 
I T T Y T D C T E S G Q N L C L C E G S N V C G Q G N K C I L -
G S D G E K N  Q C V T G E G T P K P Q S H N D G D F E E I -
P E E Y L Q.
e) Rechromatographie von Fraktion 3 (Fig. 4) lieferte ca. 150 µg eines Proteins, dessen Struktur durch Sequenzanalyse bestimmt wurde. Ihm kommt die folgende Struktur zu: 
T Y T D C T E S G Q N L C L C E G S N V C G Q G N K C I L -
G S D G E K N  Q C V T G E G T P K P Q S H N D G D F E E I -
P E E Y L Q.
f) Rechromatographie von Fraktion 4 (Fig. 5) lieferte ca. 80 µg eines Proteins, dessen Struktur durch Sequenzanalyse bestimmt wurde. Ihm kommt die folgende Struktur zu: 
I T Y T D C I E S G Q N L C L C E G S N V C G Q G N K C I L
GS D G E K N  Q C V T G E G T P K P Q S H N D G D F E E I -
P E E Y L Q.
g) Rechromatographie von Fraktion 5 (Fig. 6) lieferte ca. 40 µg eines Proteins, dessen Struktur noch nicht ermittelt wurde.

### Beispiel 8

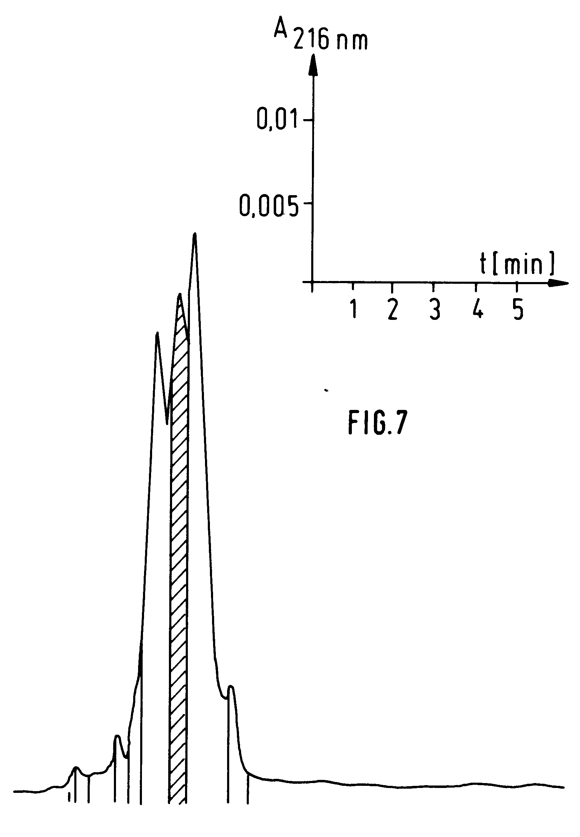

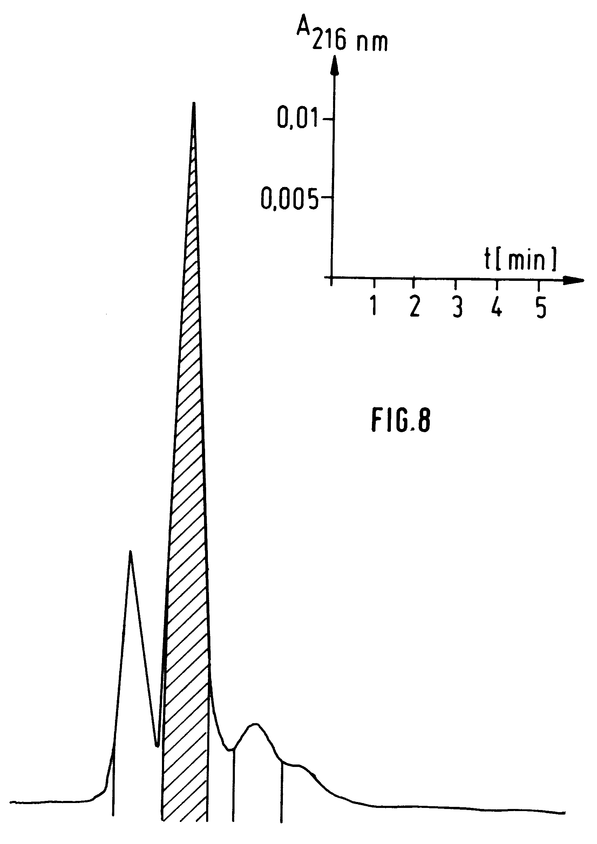

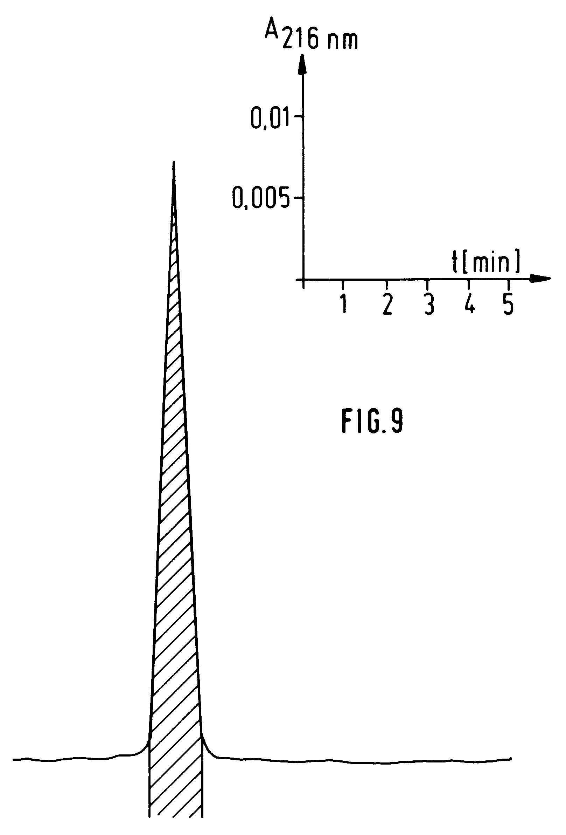

a) Man ging von einem Material aus, das gemäß Beispiel 5 durch Aufarbeitung von im Handel erhältlichen Tieren gewonnen worden war, und unterwarf es einer Vorreinigung gemäß Beispiel 7 a).
b) 1 mg der so erhaltenen Proteinfraktion wurde wie im Beispiel 7 b) mittels HPLC aufgetrennt. Die in Fig. 7 schraffierte Fraktion wurde rechromatographiert, wobei abgesehen von einer Gradientengeschwindigkeit A:B von 0,8 %/Minute die Bedingungen gleich blieben (Fig. 8). Fig. 9 zeigt das Chromatogramm des so gewonnenen analysenreinen Proteins, den die folgende durch Sequenzanalyse ermittelte Struktur zukommt:

I T Y T D C I E S G Q N L C L C E G S N V C G K G N K C I L -
G S D G  K D N Q C V T G E G T P K P Q S H N D G D F E E I -
P E E Y A Q.

| Drei- und Ein-Buchstaben-Symbole der Aminosäuren | | | |
|---|---|---|---|
| Aminosäure | Abkürzung | Aminosäure | Abkürzung |
| Alanin | Ala (A) | Phenylalanin | Phe (F) |
| Arginin | Arg (R) | Prolin | Pro (P) |
| Cystein | Cys (C) | Serin | Ser (S) |
| | | Threonin | Thr (T) |
| | | Tryptophan | Trp (W) |
| Glycin | Gly (G) | Tyrosin | Tyr (Y) |
| Histidin | His (H) | Valin | Val (V) |
| | | Asparaginsäure | Asp (D) |
| Isoleucin | Ile (I) | Asparagin | Asn (N) |
| | | Glutaminsäure | Glu (E) |
| Leucin | Leu (L) | Glutamin | Gln (Q) |
| Lysin | Lys (K) | | |
| Methionin | Met (M) | | |

## Patentansprüche

1. Polypeptid der Formel I 
1 2 3 4 5 6 7 8 9 10 11 12 13 14
H-(X)m-A-B-C-Tyr-Thr-Asp-Cys-F-Glu-Ser-Gly-Gln-Asn-Leu-
15 16 17 18 19 20 21 22 23 24 25 26 27
-Cys-Leu-Cys-Glu-Gly-Ser-Asn-Val-Cys-Gly- J -Gly-Asn-
28 29 30 31 32 33 34 35 36 37 38 49 40
-Lys-Cys-Ile-Leu-Gly-Ser-Asp-Gly- D - G -Asn-Gln-Cys-
41 42 43 44 45 46 47 48 49 50 51 52 53
-Val-Thr-Gly-Glu-Gly-Thr-Pro-Lys-Pro-Gln-Ser-His-Asn-
54 53 56 57 58 59 60 61 62 63 64 65
-Asp-Gly-Asp-Phe-Glu- E -Ile-Pro-Glu-Glu-Tyr(R)- H -
66
-Gln-(Z)n-OH

(I)
 in welcher
m= 0 - 50,
n= 0 - 100 und
R phenolischen Wasserstoff oder eine Phenolestergruppe bedeuten,
X für gleiche oder verschiedene Reste natürlich vorkommende α-Aminosäuren steht,
Z für gleiche oder verschiedene Reste naturlich vorkommender α-Aminosäuren steht und
A für Ile oder die Abwesenheit einer Aminosäure steht,
B für Ile oder Thr oder die Abwesenheit einer Aminosäure steht,
C Thr, Val, Ile, Leu oder Phe bedeutet
D Glu oder die Abwesenheit einer Aminosäure,
E Glu oder Pro,
F Thr oder Ile,
G Lys oder Lys-Asp,
H Ala oder Leu und
J Gln oder Lys bedeuten,
in der die 6 cys-Reste paarweise über Disulfid-Brücken verknüpft sind,
sowie deren physiologisch verträglichen Salze, wobei wenn
J = Gln
G ≠ Lys oder H ≠ Leu oder R ≠ Wasserstoff und falls A und B abwesend sind C ≠ Val.

2. Polypeptid nach Anspruch 1, dadurch gekennzeichnet, daß C Thr ist.

3. Polypeptid nach Anspruch 2, dadurch gekennzeichnet, daß A Ile ist.

4. Verbindung gemäß mindestens einem der Ansprüche 1 - 3, in welcher R Wasserstoff, SO₃H oder PO₃H₂ bedeuten.

5. Verbindung gemäß mindestens einem der Ansprüche 1 - 4, in welcher R für SO₃H steht.

6. Verbindung gemäß einem der Ansprüche 1 - 4, in welcher R für phenolischen Wasserstoff steht.

7. Peptidisches Spaltprodukt eines Polypeptids gemäß mindestens einem der Ansprüche 1 - 6 mit Antithrombin-Aktivität mit Ausnahme von Spaltprodukten eines Peptids der Formel:

Ile Thr Tyr Thr Asp Cys Thr Glu Ser Gly Gln Asn Leu Cys Leu Cys Glu Gly Ser Asn Val
Cys Gly Lys Gly Asn Lys Cys Ile Leu Gly Ser Asn Gly Lys Gly Asn Gln Cys Val Thr Gly
Glu Gly Thr Pro Asn Pro Gln Ser His Asn Asn Gly Asp Phe Glu Glu Ile Pro Glu Glu Tyr
Leu Gln

8. Verfahren zur Gewinnung eines gereinigten Polypeptides gemäß mindestens einem der Ansprüche 1 - 7, dadurch gekennzeichnet, daß man das Polypeptid aus Würmern der Ordnung Gnathobdellida, vorzugsweise aus Würmern der Gattung Hirudo mit Hilfe einer Kombination von Extraktionsmethoden, Fällungsmethoden, Membranfiltration und/oder chromatographischen Verfahren isoliert, eine gegebenenfalls vorhandene Phenolestergruppe R gewünschtenfalls hydrolytisch unter Bildung der phenolischen Hydroxylgruppe abspaltet, das Peptid gegebenenfalls chemisch oder enzymatisch spaltet und das erhaltene Peptid gegebenenfalls in seine physiologisch verträglichen Salze überführt.

9. Verfahren zur Herstellung eines Polypeptids der Formel 5 gemäß mindestens einem der Ansprüche 1 - 7, dadurch gekennzeichnet, daß man
a) es in an sich bekannter Weise mittels Festphasensynthese herstellt oder
b) zur Herstellung eines Polypeptids, in welchem m= 0 ist,
I. Hirudin einem zweifachen Edman-Abbau unterwirft,
II. das so erhalten Peptid mit einem Aktivester einer Aminosäure oder eines Peptids der Formel
U-Xₘ-A-B-C-OH
in welcher m, X, A,B und C wie oben definiert sind und U für eine Urethanschutzgruppe steht, umsetzt,
III. die Phenylthiocarbamoyl-Gruppe an der ε-Amino-Funktion von Lys mittels Hydrazin
IV. und die Urethan-Schutzgruppe U mit Hilfe einer Säure oder Base abspaltet, das nach a) oder b) erhaltene
Polypeptid gegebenenfalls chemisch oder enzymatisch spaltet und gegebenenfalls in sein physiologisch verträgliches Salz überführt.

10. Peptid gemäß mindestens einem der Ansprüche 1 bis 7 zur Anwendung als Arzneimittel.

11. Mittel enthaltend ein Peptid gemäß mindestens einem der Ansprüche 1 - 7 und einen pharmazeutisch unbedenklichen Träger.

## Claims

1. A polypeptide of the formula I 
1 2 3 4 5 6 7 8 9 10 11 12 13 14
H-(X)m-A-B-C-Tyr-Thr-Asp-Cys-F-Glu-Ser-Gly-Gln-Asn-Leu-·
15 16 17 18 19 20 21 22 23 24 25 26 27
-Cys-Leu-Cys-Glu-Gly-Ser-Asn-Val-Cys-Gly- J -Gly-Asn-
28 29 30 31 32 33 34 35 36 37 38 49 40
-Lys-Cys-Ile-Leu-Gly-Ser-Asp-Gly- D - G -Asn-Gln-Cys-
41 42 43 44 45 46 47 48 49 50 51 52 53
-Val-Thr-Gly-Glu-Gly-Thr-Pro-Lys-Pro-Gln-Ser-His-Asn-
54 53 56 57 58 59 60 61 62 63 64 65
-Asp-Gly-Asp-Phe-Glu- E -Ile-Pro-Glu-Glu-Tyr(R)- H -
66
-Gln-(Z)n-OH
 in which
m = 0 - 50,
n = 0 - 100 and
R denotes phenolic hydrogen or a phenolic ester group,
X represents identical or different radicals of naturally occurring α-amino acids,
Z represents identical or different radicals of naturally occurring α-amino acids and
A represents Ile or the absence of an amino acid,
B represents Ile or Thr, or the absence of an amino acid,
C denotes Thr, Val, Ile, Leu or Phe,
D denotes Glu or the absence of an amino acid,
E denotes Glu or Pro,
F denotes Thr or Ile,
G denotes Lys or Lys-Asp,
H denotes Ala or Leu and
J denotes Gln or Lys,
in which the 6 Cys radicals are linked in pairs via disulfide bridges,
or a physiologically acceptable salt thereof, where, if J = Gln, G ≠ Lys or H ≠ Leu or R ≠ hydrogen and, if A and B are absent, C ≠ Val.

2. A polypeptide as claimed in claim 1, in which C is Thr.

3. A polypeptide as claimed in claim 2, in which A is Ile.

4. A compound as claimed in any one of claims 1 - 3, in which R denotes hydrogen, SO₃H or PO₃H₂.

5. A compound as claimed in any one of claims 1 - 4, in which R represents SO₃H.

6. A compound as claimed in any one of claims 1 - 4, in which R represents phenolic hydrogen.

7. A peptidic cleavage product of a polypeptide as claimed in any one of claims 1 - 6 having antithrombin activity, with the exception of cleavage products of a peptide of the formula:

Ile Thr Tyr Thr Asp Cys Thr Glu Ser Gly Gln Asn Leu Cys Leu Cys Glu Gly Ser Asn Val
Cys Gly Lys Gly Asn Lys Cys Ile Leu Gly Ser Asn Gly Lys Gly Asn Gln Cys Val Thr Gly
Glu Gly Thr Pro Asn Pro Gln Ser His Asn Asn Gly Asp Phe Glu Glu Ile Pro Glu Glu Tyr
Leu Gln

8. A process for the isolation of a purified polypeptide as claimed in any one of claims 1 - 7, which comprises isolating the polypeptide from worms of the order Gnathobdellida, preferably from worms of the genus Hirudo, with the aid of a combination of extraction methods, precipitation methods, membrane filtration and/or chromatographic methods, if desired splitting off any phenolic ester group R which may be present by hydrolysis, to form the phenolic hydroxyl group, if appropriate cleaving the peptide chemically or enzymatically and, if appropriate, converting the resulting peptide into its physiologically acceptable salts.

9. A process for the preparation of a polypeptide of the formula I as claimed in any one of claims 1 - 7, which comprises
a) preparing it by means of solid phase synthesis in a manner which is known per se or
b) to prepare a polypeptide in which m is 0,
I. subjecting hirudin to Edman degradation twice,
II. reacting the resulting peptide with an active ester of an amino acid or of a peptide of the formula U-Xₘ-A-B-C-OH in which m, X, A, B and C are as defined above and U represents a urethane protective group,
III. splitting off the phenylthiocarbamoyl group on the ε-amino group of Lys by means of hydrazine
IV. and the urethane protective group U with the aid of an acid or base and
if appropriate cleaving the polypeptide obtained from a) or b) chemically or enzymatically, and if appropriate converting it into a physiologically acceptable salt thereof.

10. A peptide as claimed in any one of claims 1 - 7 for use as a medicine.

11. An agent containing a peptide as claimed in any one of claims 1 - 7 and a pharmaceutically acceptable excipient.

## Revendications

1. Polypeptide de formule I : 
H-(X)m-A-B-C-Tyr-Thr-Asp-Cys-F-Glu-Ser-Gly-Gln-Asn-Leu-

15 16 17 18 19 20 21 22 23 24 25 26 27
-Cys-Leu-Cys-Glu-Gly-Ser-Asn-Val-Cys-Gly- J -Gly-Asn-

28 29 30 31 32 33 34 35 36 37 38 49 40
-Lys-Cys-Ile-Leu-Gly-Ser-Asp-Gly- D - G -Asn-Gln-Cys-

41 42 43 44 45 46 47 48 49 50 51 52 53
-Val-Thr-Gly-Glu-Gly-Thr-Pro-Lys-Pro-Gln-Ser-His-Asn-

54 53 56 57 58 59 60 61 62 63 64 65
-Asp-Gly-Asp-Phe-Glu- E -Ile-Pro-Glu-Glu-Tyr(R)- H -

66
-Gln-(Z)n-OH

(I)
 dans laquelle
m = 0 - 50
n = 0 - 100 et
R désigne de l'hydrogène phénolique ou un groupe ester phénolique,
X correspond à des radicaux similaires ou différents d'α-amino acides naturels,
Z correspond à des radicaux identiques ou différents d'α-amino acides naturels et
A désigne Ile ou d'absence d'un amino acide,
B désigne Ile ou Thr ou l'absence d'un amino-acide,
C désigne Thr, Val, Ile, Leu ou Phe,
D désigne Glu ou l'absence d'un amino acide,
E désigne Glu ou Pro,
F désigne Thr ou Ile,
G désigne Lys ou Lys-Asp,
H désigne Ala ou Leu et
J désigne Gln ou Lys,
dans lesquels les 6 radicaux Cys sont reliés par paires par des ponts disulfure,
ainsi que leurs sels physiologiquement compatibles,
dans lesquels, si :
J = Gln
G est différent de Lys ou H est différent de Leu ou R n'est pas de l'hydrogène ou, au cas où A et B sont absents, C est diffèrent de Val.

2. Polypeptide selon la revendication 1, caractérisé en ce que C est Thr.

3. Polypeptide selon la revendication 2, caractérisé en ce que A est Ile.

4. Composé selon au moins l'une des revendications 1 à 3, dans lequel R désigne de l'hydrogène, un groupe SO₃H ou PO₃H₂.

5. Composé selon au moins l'une des revendications 1 à 4, dans lequel R désigne SO₃H.

6. Composéselon l'une quelconque des revendications 1 à 4, dans lequel R désigne de l'hydrogène phénolique.

7. Produit de clivage peptidique d'un polypeptide selon aumoins l'une des revendications 1 à 6 avec une activité anti-thrombine à l'exception des produits de dédoublement d'un peptide de formule :

Ile Thr Tyr Thr Asp Cys Thr Glu Ser Gly Gln Asn Leu Cys Leu Cys Glu Gly Ser Asn Val
Cys Gly Lys Gly Asn Lys Cys Ile Leu Gly Ser Asn Gly Lys Gly Asn Gln Cys Val Thr Gly
Glu Gly Thr Pro Asn Pro Gln Ser His Asn Asn Gly Asp Phe Glu Glu Ile Pro Glu Glu Tyr
Leu Gln

8. Procédé d'obtention d'un polypeptide purifié selon au moins une des revendications 1 à 7, caractérisé en ce que l'on isole le polypeptide à partir de vers de l'ordre Gnathobdellida, de préférence de vers de la souche Hirudo à l'aide d'unecombinaison de procédés d'extraction, de procédés de précipitation, de filtration sur membrane et/ou de chromatographie, on sépare un groupe d'ester phénolique R éventuellement présent le cas échéant par voie hydrolytique avec formation du groupe hydroxy phénolique, on clive le peptide éventuellement par voie chimique ou enzymatique et l'on transforme le peptide obtenu éventuellement en ses sels physiologiquement compatibles.

9. Procédé de préparation d'un polypeptide de formule I selon au moins une des revendications 1 à 7, caractérisé en ce que :
a) le polypeptide est préparé de manière connue en soi par synthèse en phase solide ou
b) pour la préparation d'un polypeptide dans lequel m= 0
I. on soumet de l'hirudine à une double décomposition d'Edman,
II. on fait réagir le peptide ainsi obtenu avec un ester actif d'un amino-acide ou d'un peptide de formule :
U-(X)ₘ-A-B-C-OH,
dans laquelle m, X et A, B, C sont tels que définis ci-dessus et U désigne un groupe de protection de type uréthane instable dans les acides et les bases,
III. on sépare le groupe phénylthiocarbamoyle sur la fonction ε-amino du Lys à l'aide d'hydrazine et
IV. le groupe de protection de type uréthane U à l'aide d'un acide ou d'une base, et l'on sépare le polypeptide obtenu selon a) ou b) éventuellement par voie chimique ou enzymatique et on le transforme éventuellement en son sel compatible physiologiquement.

10. Peptide selon l'une quelconque des revendications 1 à 7 pour application comme médicament.

11. Agent contenant un peptide selon au moins l'une des revendications 1 à 7 et un véhicule pharmaceutiquement acceptable.
